(19) Europäisches Patentamt
European Patent Office
Office européen des brevets

(11) EP 2 509 934 B1

(12) # EUROPEAN PATENT SPECIFICATION

(45) Date of publication and mention of the grant of the patent:
**19.02.2014 Bulletin 2014/08**

(21) Application number: **10836586.7**

(22) Date of filing: **08.12.2010**

(51) Int Cl.:
***C07C 2/66*** *(2006.01)*

(86) International application number:
**PCT/US2010/059383**

(87) International publication number:
**WO 2011/071977 (16.06.2011 Gazette 2011/24)**

(54) **AROMATIC ALKYLATION PROCESS**

VERFAHREN ZUR AKLYLIERUNG VON AROMASTOFFEN

PROCÉDÉ D'ALKYLATION AROMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2009 US 653043**

(43) Date of publication of application:
**17.10.2012 Bulletin 2012/42**

(73) Proprietor: **Saudi Basic Industries Corporation
Riyadh 11422 (SA)**

(72) Inventors:
- **GHOSH, Ashim, Kumar
Houston, TX 77059 (US)**
- **HARVEY, Pamela
Missouri City, TX 77489 (US)**
- **KULKARNI, Neeta
Houston, TX 77062 (US)**

(74) Representative: **Vroemen, Maurice et al
SABIC Intellectual Property Group
P.O.Box 3008
6160 GA Geleen (NL)**

(56) References cited:
**US-A- 3 702 886**
**US-A- 4 250 345**
**US-A- 5 178 748**
**US-A1- 2006 252 633**
**US-B2- 7 060 864**
**US-B2- 7 084 318**
**US-B2- 7 186 872**
**US-B2- 7 304 194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 509 934 B1

**Description**

BACKGROUND OF THE INVENTION

FIELD OF THE INVENTION: The invention relates generally to a process for the alkylation of aromatic compounds, e.g., toluene methylation, with a zeolite catalyst, e.g., an aluminosilicate zeolite which has been modified to be a shape selective catalyst, in the presence of hydrogen and steam, said process relating to introduction of the alkylating agent and hydrogen in a certain order of introduction and within a certain temperature range.

DESCRIPTION OF THE PRIOR ART: Toluene methylation (TM) is a catalytic reaction of toluene with methanol to produce xylenes as shown below:

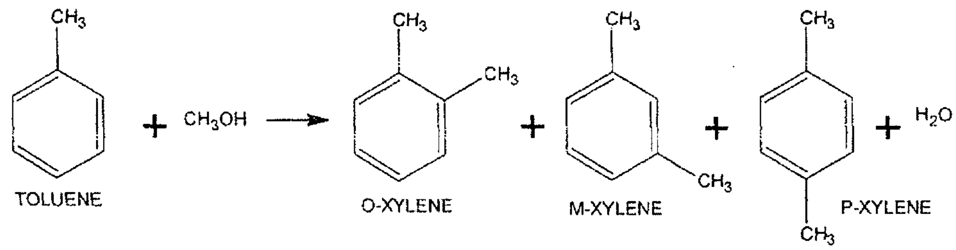

Toluene methylation is an equimolar reaction, i.e., one mole of toluene reacts with one mole of methanol. In addition to the methylation of toluene, many competing side reactions can occur. Methanol may react with itself to form olefins. Toluene can be over-alkylated to form $C_{9+}$ aromatics. To reduce these side reactions, the toluene:methanol mole ratio may be greater than 1:1 or in the range from 2:1 to 20:1.

Zeolites which are crystalline solids made up of aluminum-substituted $SiO_4$ tetrahedral units joined together to form different ring and cage structures into a crystalline framework can be used as catalyst for toluene methylation. The physical structure of zeolite is very porous with a large internal and external surface area. Zeolites can be shape-selective catalysts due to steric and electronic effects. Shape selective properties can be obtained by modifying the zeolite, e.g., narrowing zeolite pore opening size, inactivation of the external surface of the zeolite or altering zeolite acidity. Deposition of certain compounds or elements on the zeolite can make it more shape selective, e.g., compounds containing iron, zinc, phosphorous, rare earth metal oxides, etc.

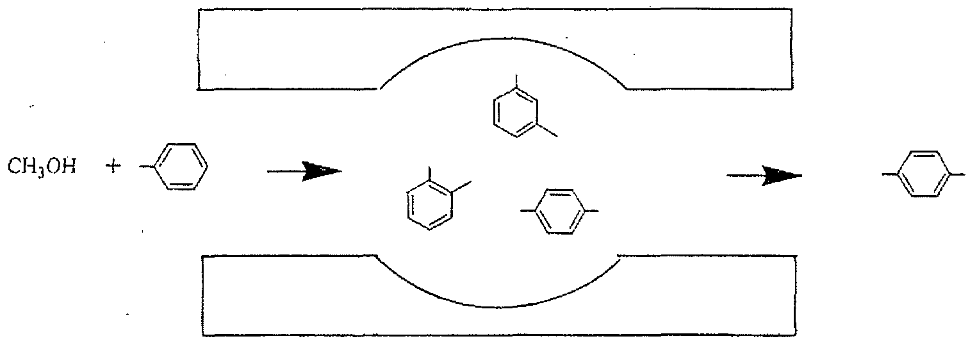

In the synthesis of p-xylene by methylation of toluene, the conversion of toluene and the selectivity of p-xylene, i.e., concentration of p-xylene in the xylene isomers are of commercial importance. Para-xylene (PX) is of particular value as a large volume chemical intermediate in a number of applications being useful in the manufacture of terephtalates which are intermediates for the manufacture of PET. It would be advantageous for a process to produce p-xylene at concentrations of at least 85% or at least 90%.

U.S. Patent no. 4,152,364 discloses a process for the selective production ofpara-xylene by methylation of toluene in the presence of a catalyst of zeolite which has been modified or treated with a phosphorus compound. Methylation of toluene is by contacting toluene with methanol at a temperature between about 250°C and about 750°C, preferably between about 400°C and about 700°C. There is no disclosure that the toluene and methanol are introduced into the reactor separately or at different temperatures.

U.S. Patent no. 4,250,345 discloses a process for the selective production of para-xylene by methylation of toluene in the presence of a catalyst of zeolite which has been modified or treated with a phosphorus compound. Methylation of toluene is by contacting toluene with methanol at a temperature between about 250°C and about 750°C, preferably between about 500°C and about 700°C. There is no disclosure that the toluene and methanol are introduced

into the reactor separately or at different temperatures.

U.S. Patent no. 7,084,318 discloses a method of preparing a xylene product with a phosphorus-treated ZSM-5 catalyst having a startup procedure of introducing toluene, methanol and hydrogen at a liquid hourly space velocity (LHSV) of more than 5 $hr^{-1}$ at a temperature of 200°C or above, operating at these conditions for a half hour to twenty hours and then reducing the LHSV and increasing the temperature to 500°C - 700°C. There is no disclosure that the toluene and methanol are introduced into the reactor separately or at different temperatures.

A process which would increase p-xylene concentration in the mixed-xylene product stream and which would decrease catalyst deactivation and maintain toluene conversion to increase time on stream would be advantageous.

## SUMMARY OF THE INVENTION

The present invention is for a method of alkylation of an aromatic comprising:

a) contacting an aromatic compound, an alkylating agent; hydrogen, an inert gas or a mixture thereof; and water with a shape-selective zeolite catalyst in a reactor in the steps of:

1) introducing hydrogen, an inert gas or a mixture thereof to the reactor at a reactor temperature of about 200°C to less than about 400°C; and
2) introducing an alkylating agent to the reactor at a reactor temperature of more than about 400°C to about 700°C;

b) withdrawing an alkylated aromatic from the reactor.

Contacting the aromatic compound; the alkylating agent; hydrogen, an inert gas or a mixture thereof; and water with a shape-selective zeolite catalyst in a reactor may comprise the steps of:

1) heating the reactor to a temperature of about 200°C to less than about 400°C;
2) introducing hydrogen, an inert gas or a mixture thereof to the reactor at a reactor temperature of about 200°C to less than about 400°C;
3) heating the reactor to a temperature of more than about 400°C to about 700°C; and
4) introducing an alkylating agent to the reactor at a reactor temperature of more than about 400°C to about 700°C;
wherein the aromatic compound is introduced to the reactor at step 2), step 3) or step 4) and the water is introduced to the reactor at step 2), step 3) or step 4).

## BRIEF DESCRIPTION OF THE DRAWINGS

Figure 1 shows a graph of hours on stream v. per cent toluene conversion for Examples 1-7 with hydrogen, steam, toluene and methanol each being introduced into the reactor at a reactor temperature of 200°C or of 480°C.

## DESCRIPTION OF EMBODIMENTS OF THE INVENTION

Toluene methylation is known to occur over zeolite or zeolite-type catalysts, in particular, ZSM-5-type zeolite catalysts. Generally, a thermodynamic equilibrium mixture of ortho (o)-, meta (m)- and para (p)-xylenes is formed from the methylation of toluene. Thermodynamic equilibrium compositions of o-, m-, and p-xylenes may be around 25, 50 and 25 mole%, respectively, at a reaction temperature of about 500 °C. Such toluene methylation may occur over at wide range of temperatures, however.

A high purity grade (99+%) p-xylene is desirable for its oxidation to terephthalic acid process. Thus, an increased concentration of p-xylene over equilibrium is desirable.

Zeolite is a crystalline hydrated aluminosilicate that may also contain other metals, such as sodium, calcium, barium, and potassium, and that has ion exchange properties (Encarta® World English Dictionary [North American Edition] © & (P) 2001 Microsoft Corporation). Examples of zeolites are ZSM-5, ZSM-11, ZSM-5/ZSM-11 intermediate, ZSM-12, ZSM-21, ZSM-22, ZSM-23, ZSM-35, ZSM-38, ZSM-48, ZSM-50, MCM-22, Zeolite L, Zeolite Beta and Mordenite which are known in the art. The present invention incorporates modification of zeolites to be shape selective catalysts.

ZSM-5 zeolite is a porous material containing intersecting two-dimensional pore structure with 10-membered oxygen rings. Zeolites with such 10-membered oxygen ring pore structures are often classified as medium-pore zeolites. As used herein, the expression "ZSM-5-type" is meant to refer to those zeolites that are isostructurally the same as ZSM-5 zeolites. Additionally, the expressions "ZSM-5" and "ZSM-5-type" may also be used herein interchangeably to

encompass one another and should not be construed in a limiting sense.

ZSM-5 zeolite catalysts and their preparation are described in U.S. Patent No. 3,702,886. In the present invention, the ZSM-5 zeolite catalyst may include those having a silica/alumina molar ratio of 200 or higher, more particularly from about 250 to about 500 prior to modification. The starting ZSM-5 may be an $NH_4^+$ or $H^+$ form and may contain traces of other cations. Modification of ZSM-5-type zeolite with phosphorus-containing compounds has been shown to provide shape selective properties to the catalyst, yielding significantly greater amounts of p-xylene than the thermodynamic equilibrium value when used in toluene methylation compared to unmodified catalysts. Such modification has been shown to provide selectivity for p-xylenes of greater than 80%.

The catalysts of the present invention are shape selective zeolite catalysts. One example of a shape selective zeolite catalyst is a zeolite modified by treating with phosphorus-containing compounds including, but are not limited to, phosphonic, phosphinous, phosphorus and phosphoric acids, salts and esters of such acids and phosphorous halides. In particular, phosphoric acid ($H_3PO_4$) and ammonium hydrogen phosphate ($(NH_4)_2HPO_4$) may be used as the phosphorus-containing compound to provide a catalyst for toluene methylation with shape selective properties to provide increased p-xylene selectivity. Such modified catalysts may contain phosphorus (P) in an amount of from about 0.01 to about 0.15 g P/g zeolite, more particularly from about 0.02 to about 0.13 g P/g zeolite, and more particularly from about 0.07 g P/g zeolite to about 0.12 g P/g zeolite, and still more particularly from about 0.09 g P/g zeolite to about 0.11 g P/g zeolite. After phosphorus treatment, the phosphorus-treated zeolite may be dried. One example of the zeolite is a ZSM-5-type.

The P-modified ZSM-5 catalyst may be contacted with an appropriate feed of an aromatic hydrocarbon and an alkylating agent under alkylation reaction conditions to carry out aromatic alkylation. One particular application of the catalyst is for use in toluene methylation utilizing a toluene/methanol feed.

The process of the present invention may also have a gas feed. The feed gas may include hydrogen or an inert gas. Examples of inert gases are nitrogen, carbon monoxide, carbon dioxide and the noble gases (helium, neon, argon, krypton, xenon and radon). The feed gas may be hydrogen, an inert gas or mixtures thereof.

As used herein, the expression "alkylation feed" is meant to encompass the aromatic compound and the alkylating agent. As used herein, the expression "methylation feed" is meant to encompass the feed of toluene and methanol.

In addition to any feed gas, water that may be in the form of steam may also be introduced into the reactor. The water or steam used for the alkylation reaction may be introduced with or without hydrogen or inert gas as cofeed with the alkylation feed to the reactor during the start up of the alkylation reaction, or it may be introduced subsequent to initial start up. In either case, liquid water may be added and vaporized prior to its mixing with cofeed gas (if any) and the alkylation feed. The use of water cofeed is described in U.S. Patent No. 7,060,864 issued June 13, 2006, and entitled "Toluene Methylation Process," and in U.S. Patent No. 7,186,872 issued March 6, 2007, as a continuation-in-part application entitled "Toluene Methylation Process with Increased Methanol Selectivity". Steam may be used to heat the reactor while steaming the catalyst for about 1 to about 16 hours.

The reactor pressure for toluene methylation or other aromatic alkylation may vary, but typically ranges from about 10 to about 1000 psig. Reactor temperatures may vary, but typically range from about 400 to about 700°C. Upon introduction of feed into the reactor, the catalyst bed temperature may be adjusted to a selected reaction temperature to effect a desired conversion. The temperature may be increased gradually at a rate of from about 1°C/min to about 10°C/min to provide the desired final reactor temperature. As used in the examples, reactor temperature refers to the temperature as measured at the inlet of the catalyst bed of the reactor.

The reaction may be carried out in a variety of different reactors that are commonly used for carrying out aromatic alkylation reactions. Single or multiple reactors in series and/or parallel are suitable for carrying out the aromatic alkylation. Alkylating agent, hydrogen, water and/or aromatic may be added to the product stream entering the second and subsequent reactors when using multiple reactors in series.

The aromatic alkylation process of the present invention involves introducing hydrogen, an inert gas or a mixture thereof into a reactor or reaction zone at a temperature less than reaction temperature. In one embodiment hydrogen is introduced into a reactor or reaction zone which has been heated to a temperature less than reaction temperature. In another embodiment hydrogen is introduced into a reactor or reaction zone while heating to a temperature less than reaction temperature. The temperature less than reaction temperature may be a temperature of about 200°C to less than about 400°C. The temperature may be in the range from about 200°C to about 250°C. One specific example of the temperature is about 200°C. Optionally, water, either as liquid water or as steam, and/or an aromatic, such as toluene, may also be introduced into the reactor or reaction zone at a temperature less than reaction temperature. Hydrogen and water may be mixed with each other or either or both may be mixed with the aromatic before introduction into the reactor or reaction zone or hydrogen, water and aromatic may be introduced separately into the reactor or reaction zone. No significant amount of alkylating agent is introduced into the reactor or reaction zone when the temperature is less than reaction temperature. With the context of this invention, "no significant amount of alkylating agent" is meant to mean more than 1% by weight.

The aromatic alkylation process of the present invention also involves introducing an alkylating agent into the reactor or reaction zone at a temperature within the range of reaction temperature. In one embodiment the alkylating agent is introduced into a reactor or reaction zone which has been heated to a temperature of more than about 400°C to about 700°. The temperature may be in the range from about 400°C to about 500°C. One specific example of the temperature is about 480°C. Optionally, water, either as liquid water or as steam, and/or an aromatic, such as toluene, may also be introduced into the reactor or reaction zone at a temperature within the range of reaction temperature. The aromatic and the alkylating agent may be mixed with each other or either or both may be mixed with the water before introduction into the reactor or reaction zone or the alkylating agent, water and aromatic may be introduced separately into the reactor or reaction zone.

In the aromatic alkylation process of the present invention hydrogen, an inert gas or a mixture thereof is introduced into a reactor or reaction zone at a temperature less than reaction temperature, e.g., about 200°C to less than about 400°C, after which an alkylating agent is introduced into the reactor or reaction zone at a temperature within the range of reaction temperature, e.g., more than about 400°C to about 700°. The water, either as liquid water or as steam, and the aromatic introduced into the reactor or reaction zone can be at a temperature less than reaction temperature or at a temperature within the range of reaction temperature and can be introduced into the reactor before, as or after the hydrogen, inert gas or a mixture thereof is introduced, before, as or after the alkylating agent is introduced or between introduction of the hydrogen, inert gas or a mixture thereof and the alkylating agent. In one embodiment of the present invention, the alkylating agent, water, either as liquid water or as steam, and the aromatic are introduced into the reactor or reaction zone at a temperature within the range of reaction temperature, e.g., more than about 400°C to about 700°C. In another embodiment of the present invention, hydrogen and water, either as liquid water or as steam, are introduced into the reactor or reaction zone at a temperature less than reaction temperature, e.g., about 200°C to less than about 400°C and the alkylating agent and the aromatic are introduced into the reactor or reaction zone at a temperature within the range of reaction temperature, e.g., more than about 400°C to about 700°C. In another embodiment of the present invention, hydrogen, the aromatic and water, either as liquid water or as steam, are introduced into the reactor or reaction zone at a temperature less than reaction temperature, e.g., about 200°C to less than about 400°C.

The invention having been generally described, the following examples are given as particular embodiments of the invention and to demonstrate the practice and advantages thereof. It is understood that the examples are given by way of illustration and are not intended to limit the invention, the specification or the claims in any manner.

As used herein, catalytic activity can be expressed as the % moles of the toluene converted with respect to the moles of toluene fed and can be defined by the following formulas:

$$\text{Mole\% Toluene Conversion} = [(T_i - T_o)/T_i] \times 100 \qquad (1)$$

where, $T_i$ is the number of moles of toluene fed and $T_o$ is the number of moles toluene unreacted. As used herein, selectivity for mixed xylenes may be expressed as:

$$\text{Mole\% Mixed Xylene Selectivity} = [X_{tx}/(T_i - T_o)] \times 100 \qquad (2)$$

where, $X_{tx}$ is the number of moles of mixed (o-, m- or p-) xylenes in the product.

As used herein, selectivity for p-xylene may be expressed as:

$$\text{Mole\% p-Xylene Selectivity} = (X_p/X_{tx}) \times 100 \qquad (3)$$

where $X_p$ is the number of moles of p-xylene.

As used herein, para-xylene yield may be expressed as:

$$\text{Mole\% p-Xylene Yield} = (\text{Mole \% Toluene Conversion}) \times \qquad (4)$$

(Mole % Mixed Xylene Selectivity) x (Mole % p-Xylene Selectivity) x $10^{-4}$

As used herein, methanol conversion may be expressed as:

$$\text{Mole \% Methanol Conversion} = [(M_i - M_o)/M_i] \times 100 \qquad (5)$$

where, $M_i$ is the number of moles of methanol fed and $M_o$ is the number of moles methanol unreacted.

As used herein, methanol selectivity for toluene methylation may be expressed as:

$$\text{Mole\% Methanol Selectivity} = [X_{tx}/(M_i\text{-}M_o)] \times 100 \qquad (6)$$

where, $X_{tx}$ is the number of moles of mixed (o-, m- or p-) xylenes, $M_i$ is the number of moles of methanol fed and $M_o$ is the number of moles of unreacted methanol.

EXAMPLES

Catalyst Preparation

A $NH_4$-ZSM-5 zeolite powder having a $SiO_2/Al_2O_3$ mole ratio of about 275 was treated with aqueous $H_3PO_4$ and was then calcined at a temperature of about 530 °C. The phosphorus treated ZSM-5 zeolite was crushed into fine powder and was then combined with alumina as binder and extruded into 1/16-inch diameter cylindrical-shaped extrudates. The bound zeolite extrudates were then calcined or heated to a temperature of 530 °C to form Catalyst A that contained about 20 wt% alumina binder and approximately 7.4 wt% phosphorus by total weight of catalyst.

Catalyst A was used in all examples to demonstrate the invention. In carrying out a reaction of toluene with methanol in presence of cofeed steam and hydrogen, in each example, a catalyst charge of about 5.4 ml (catalyst size: 20-40 mesh) loaded in a SS-316 tube (OD ¾-inch, ID) reactor. Catalyst was dried by slowly raising the catalyst bed temperature (about 5°C/min) to 200°C under hydrogen flow (50 cc/min) for at least one hour. Catalyst was then steamed by introducing water vapor (2.2 mmole/min) with a carrier gas of $H_2$ (459 cc/min) at 200°C overnight. In examples 1-7, after steaming the catalyst toluene, methanol, water and hydrogen were introduced into the reactor at different conditions (temperature and order of introduction) to effect the catalyst performance particularly catalyst deactivation resulting in the yield of p-xylene product. Toluene, methanol, water and hydrogen were fed to the reactor via a preheater at 200°C so that water, methanol and toluene where vaporized and mixed before entering the reaction zone.

COMPARATIVE EXAMPLE I

As described above, catalyst was steamed at 200°C overnight and then toluene, methanol, water, and hydrogen were introduced into the reactor at 200°C at the rates shown below. The reactor temperature was then increased and catalyst bed inlet temperature was adjusted to 485°C which was maintained constant during the test run. The reactor streams were analyzed to calculate conversion, selectivity and p-xylene yield shown in Table 1.
Catalyst charge 5.40 ml (4.19 g)
Catalyst steamed at 200°C overnight

Feed introduction:

At 200°C, $H_2$ 420 cc/min, $H_2O$ 0.029 g/min, Premixed toluene and methanol (3.0 molar ratio) 0.16-0.17 g/min
Reactor inlet pressure 20 psig

Table 1

| Time on stream (h) | Toluene Conversion, mole% | Mixed Xylene Selectivity, mole% | Para-Xylene Selectivity, mole% | Para-Xylene Yield, mole% |
|---|---|---|---|---|
| 23.52 | 14.26 | 97.06 | 95.59 | 13.23 |
| 31.08 | 13.25 | 96.56 | 94.63 | 12.11 |
| 47.27 | 11.53 | 96.39 | 94.17 | 10.47 |
| 54.85 | 11.54 | 96.20 | 93.87 | 10.42 |
| 71.92 | 10.68 | 96.19 | 93.41 | 9.60 |

(continued)

| Time on stream (h) | Toluene Conversion, mole% | Mixed Xylene Selectivity, mole% | Para-Xylene Selectivity, mole% | Para-Xylene Yield, mole% |
|---|---|---|---|---|
| 78.77 | 11.02 | 96.27 | 93.27 | 9.89 |
| 143.82 | 9.39 | 95.62 | 92.31 | 8.29 |
| 150.65 | 8.60 | 95.40 | 92.09 | 7.56 |
| 167.63 | 8.35 | 95.60 | 91.94 | 7.34 |
| 175.02 | 8.38 | 95.37 | 91.87 | 7.34 |
| 191.40 | 7.96 | 95.34 | 91.87 | 6.97 |
| 199.05 | 8.00 | 95.36 | 91.69 | 6.99 |
| 215.13 | 7.73 | 95.37 | 91.42 | 6.74 |
| 239.05 | 7.53 | 95.29 | 91.43 | 6.56 |
| 246.02 | 7.36 | 95.33 | 91.38 | 6.41 |

COMPARATIVE EXAMPLE 2

As described earlier, catalyst was steamed at 200°C overnight in presence of steam and hydrogen. Flows of hydrogen and water were discontinued after steaming the catalyst and reactor temperature was increased to about 480°C at which time hydrogen, water, and premixed toluene and methanol were introduced into the reactor at the rates shown below. The catalyst bed inlet temperature was adjusted to 485°C which was maintained constant during the test run. The reactor streams were analyzed to calculate conversion, selectivity and p-xylene yield shown in Table 2.
Catalyst charge 5.40 ml (4.19 g)
Catalyst steamed at 200°C overnight

Feed introduction:

At 480°C, $H_2$ 420 cc/min, $H_2O$ 0.029 g/min, Premixed toluene and methanol (3.0 molar ratio) 0.16-0.17g/min
Reactor inlet pressure 20 psig

Table 2

| Time on stream (h) | Toluene Conversion, mole% | Mixed Xylene Selectivity, mole% | Para-Xylene Selectivity, mole% | Para-Xylene Yield, mole% |
|---|---|---|---|---|
| 2.42 | 14.72 | 97.37 | 95.69 | 13.72 |
| 18.45 | 12.72 | 96.43 | 94.57 | 11.60 |
| 26.42 | 11.15 | 96.13 | 93.61 | 10.03 |
| 42.83 | 10.90 | 96.25 | 93.49 | 9.81 |
| 49.62 | 10.56 | 95.92 | 92.71 | 9.39 |
| 66.35 | 9.80 | 96.01 | 92.73 | 8.72 |
| 71.40 | 10.39 | 96.03 | 92.69 | 9.25 |
| 162.57 | 7.76 | 95.16 | 90.60 | 6.69 |
| 170.12 | 7.32 | 95.13 | 90.31 | 6.29 |
| 186.63 | 7.09 | 95.08 | 89.97 | 6.07 |
| 194.35 | 7.08 | 95.17 | 90.01 | 6.06 |
| 210.63 | 7.31 | 95.22 | 90.19 | 6.28 |
| 218.02 | 7.08 | 95.12 | 90.08 | 6.07 |

(continued)

| Time on stream (h) | Toluene Conversion, mole% | Mixed Xylene Selectivity, mole% | Para-Xylene Selectivity, mole% | Para-Xylene Yield, mole% |
|---|---|---|---|---|
| 234.57 | 6.99 | 95.08 | 90.05 | 5.98 |
| 241.35 | 6.94 | 94.86 | 89.65 | 5.90 |

COMPARATIVE EXAMPLE 3

After steaming the catalyst, hydrogen, water and methanol were introduced into the reactor at 200°C at the rates shown below. The reactor temperature was then increased to 480°C at which time toluene was introduced into reactor (that is, methanol was discontinued and premixed toluene and methanol started), and the catalyst bed inlet temperature was adjusted to 485°C which was maintained constant during the test run and reactor streams were analyzed to calculate conversion, selectivity and p-xylene yield shown in Table 3.
Catalyst charge 5.40 ml (4.19 g)
Catalyst steamed at 200°C overnight

Feed introduction:

At 200°C, $H_2$ 420 cc/min, $H_2O$ 0.029 g/min, Methanol 0.16-0.17 g/min
Reactor inlet pressure 20 psig
At 480°C, methanol flow was replaced by premixed toluene and methanol (3:0 molar ratio) 0.16-0.17 g/min

Table 3

| Time on stream (h) | Toluene Conversion, mole% | Mixed Xylene Selectivity, mole% | Para-Xylene Selectivity, mole% | Para-Xylene Yield, mole% |
|---|---|---|---|---|
| 29.58 | 16.71 | 95.88 | 92.79 | 14.87 |
| 46.75 | 14.34 | 95.98 | 92.58 | 12.74 |
| 53.25 | 13.51 | 95.76 | 92.26 | 11.94 |
| 71.00 | 12.89 | 95.48 | 92.12 | 11.34 |
| 77.42 | 12.88 | 95.45 | 92.09 | 11.32 |
| 142.52 | 11.67 | 95.56 | 91.69 | 10.23 |
| 149.67 | 10.98 | 95.45 | 91.39 | 9.58 |
| 166.67 | 10.53 | 95.32 | 91.25 | 9.16 |
| 173.35 | 10.32 | 95.38 | 91.05 | 8.96 |
| 191.17 | 9.97 | 94.69 | 90.88 | 8.58 |
| 197.52 | 10.04 | 95.23 | 90.84 | 8.69 |

COMPARATIVE EXAMPLE 4

After steaming the catalyst, hydrogen flow discontinued but water flow (rate shown below) was maintained through the catalyst bed, and the reactor temperature was then increased to 480°C. Hydrogen and premixed toluene and methanol were introduced into reactor at 480°C, and the catalyst bed inlet temperature was adjusted to 485°C which was maintained constant during the test run and reactor streams were analyzed to calculate conversion, selectivity and p-xylene shown in Table 4.
Catalyst charge 5.40 ml (4.19 g)
Catalyst steamed at 200°C overnight

Feed introduction:

At 200°C, $H_2O$ 0.03 g/min

At 480°C, $H_2$, toluene and methanol were introduced into reactor at rates below.
$H_2$ 420 cc/min
Premixed toluene and methanol (molar ratio 3.0) 0.16-0.17 g/min
Reactor inlet pressure 20 psig

Table 4

| Time on stream (h) | Toluene Conversion, mole% | Mixed Xylene Selectivity, mole% | Para-Xylene Selectivity, mole% | Para-Xylene Yield, mole% |
|---|---|---|---|---|
| 19.25 | 13.80 | 97.12 | 95.59 | 12.81 |
| 26.82 | 13.16 | 96.88 | 95.22 | 12.14 |
| 43.43 | 12.55 | 96.85 | 94.97 | 11.54 |
| 50.70 | 12.94 | 96.97 | 95.00 | 11.92 |
| 67.20 | 12.41 | 96.72 | 94.89 | 11.39 |
| 73.73 | 12.27 | 96.88 | 94.85 | 11.27 |
| 139.47 | 12.43 | 96.86 | 94.83 | 11.42 |
| 146.45 | 11.81 | 96.42 | 94.64 | 10.78 |
| 163.10 | 11.61 | 96.74 | 94.57 | 10.62 |
| 170.75 | 11.11 | 96.26 | 94.23 | 10.08 |
| 187.17 | 10.03 | 96.77 | 94.36 | 9.16 |

EXAMPLE 5

After steaming the catalyst, hydrogen, steam and toluene were introduced into the reactor at 200°C at the rates shown below. The reactor temperature was increased to 480°C at which time methanol was introduced into reactor (that is, toluene flow was discontinued and premixed toluene and methanol started), and the catalyst bed inlet temperature was adjusted to 485°C which was maintained constant during the test run and reactor streams were analyzed to calculate conversion, selectivity and p-xylene yield shown in Table 5.
Catalyst charge 5.40 ml (4.19 g)
Catalyst steamed at 200°C overnight

Feed introduction:

At 200°C, $H_2$ 420 cc/min, $H_2O$ 0.029 g/min, Toluene 0.16-0.17 g/min
Reactor inlet pressure 20 psig
At 480°C, toluene flow was replaced by premixed toluene and methanol (3:0 molar ratio) 0.16-0.17 g/min

Table 5

| Time on stream (h) | Toluene Conversion, mole% | Mixed Xylene Selectivity, mole% | Para-Xylene Selectivity, mole% | Para-Xylene Yield, mole% |
|---|---|---|---|---|
| 23.53 | 16.52 | 97.52 | 96.49 | 15.54 |
| 43.25 | 15.81 | 97.32 | 95.94 | 14.76 |
| 50.55 | 14.63 | 97.04 | 95.31 | 13.53 |
| 67.25 | 14.10 | 96.88 | 95.09 | 12.99 |
| 74.38 | 14.20 | 96.96 | 95.01 | 13.08 |
| 139.40 | 13.18 | 96.71 | 94.73 | 12.07 |
| 146.80 | 12.74 | 96.51 | 94.49 | 11.62 |
| 163.23 | 12.40 | 96.52 | 94.37 | 11.29 |

(continued)

| Time on stream (h) | Toluene Conversion, mole% | Mixed Xylene Selectivity, mole% | Para-Xylene Selectivity, mole% | Para-Xylene Yield, mole% |
|---|---|---|---|---|
| 170.47 | 12.79 | 96.63 | 94.44 | 11.67 |
| 186.95 | 12.09 | 96.48 | 94.22 | 10.99 |
| 194.75 | 12.09 | 96.39 | 94.30 | 10.99 |
| 211.13 | 11.66 | 96.38 | 94.09 | 10.57 |
| 218.77 | 11.49 | 96.42 | 93.96 | 10.41 |
| 235.17 | 11.60 | 96.39 | 93.88 | 10.50 |
| 242.43 | 11.21 | 96.31 | 93.81 | 10.13 |

EXAMPLE 6

After steaming the catalyst at 200°C, only hydrogen was introduced into the reactor at 200°C at the rate shown below. The reactor temperature was then increased to 480°C at which time $H_2O$ and premixed toluene and methanol were introduced into reactor, and the catalyst bed inlet temperature was adjusted to 485°C and was maintained constant during the test run and reactor streams were analyzed to calculate conversion, selectivity and p-xylene yield shown in Table 6.
Catalyst charge 5.40 ml (4.19 g)
Catalyst steamed at 200°C overnight

Feed introduction:

At 200°C, $H_2$ 420 cc/min
At 480°C, steam, toluene and methanol were introduced into reactor at rates below.
$H_2O$ 0.029 g/min, Premixed toluene and methanol (molar ratio 3.0) 0.16-0.17 g/min
Reactor inlet pressure 20 psig

Table 6

| Time on stream (h) | Toluene Conversion, mole% | Mixed Xylene Selectivity, mole% | Para-Xylene Selectivity, mole% | Para-Xylene Yield, mole% |
|---|---|---|---|---|
| 23.25 | 16.53 | 96.81 | 93.91 | 15.03 |
| 40.25 | 16.17 | 95.82 | 93.61 | 14.50 |
| 65.42 | 15.17 | 96.08 | 93.22 | 13.59 |
| 71.33 | 14.48 | 96.13 | 93.04 | 12.95 |
| 136.67 | 13.75 | 95.61 | 92.66 | 12.18 |
| 160.42 | 12.75 | 95.25 | 92.22 | 11.20 |
| 166.00 | 12.96 | 96.02 | 92.27 | 11.48 |
| 184.83 | 13.34 | 95.53 | 92.42 | 11.78 |
| 191.73 | 12.68 | 95.83 | 92.26 | 11.21 |
| 209.07 | 12.09 | 95.13 | 91.93 | 10.57 |
| 215.93 | 12.18 | 95.84 | 91.96 | 10.73 |
| 232.47 | 11.93 | 95.68 | 91.89 | 10.49 |
| 239.65 | 11.76 | 94.55 | 91.52 | 10.18 |

EXAMPLE 7

After steaming the catalyst at 200°C, hydrogen and steam were introduced into the reactor at 200°C at the rate shown below. The reactor temperature was then increased to 480°C at which time premixed toluene and methanol were introduced into reactor, and the catalyst bed inlet temperature was adjusted to 485°C and was maintained constant during the test run and reactor streams were analyzed to calculate conversion, selectivity and p-xylene yield shown in Table 7.
Catalyst charge 5.40 ml (4.19 g)
Catalyst steamed at 200°C overnight

Feed introduction:

At 200°C, $H_2$ 420 cc/min, $H_2O$ 0.029 g/min
Reactor inlet pressure 20 psig
At 480°C, toluene and methanol were introduced into reactor at rates below. Premixed toluene and methanol (molar ratio 3.0) 0.16-0.17 g/min
Adjusted and maintained catalyst bed inlet temperature at 485°C

Table 7

| Time on stream (h) | Toluene Conversion, mole% | Mixed Xylene Selectivity, mole% | Para-Xylene Selectivity, mole% | Para-Xylene Yield, mole% |
|---|---|---|---|---|
| 19.48 | 16.18 | 97.50 | 96.14 | 15.17 |
| 26.65 | 15.98 | 97.29 | 95.86 | 14.90 |
| 43.23 | 15.13 | 97.16 | 95.62 | 14.06 |
| 50.87 | 14.63 | 96.90 | 95.37 | 13.52 |
| 67.33 | 14.26 | 97.07 | 95.26 | 13.19 |
| 73.87 | 14.45 | 96.90 | 95.12 | 13.32 |
| 139.37 | 12.90 | 96.65 | 94.70 | 11.81 |
| 146.27 | 12.67 | 96.76 | 94.42 | 11.58 |
| 163.30 | 12.18 | 96.63 | 94.36 | 11.11 |
| 171.00 | 11.74 | 96.57 | 94.25 | 10.69 |
| 187.22 | 11.32 | 96.24 | 94.10 | 10.25 |
| 194.57 | 11.14 | 96.72 | 94.13 | 10.14 |
| 211.35 | 11.23 | 96.48 | 94.08 | 10.19 |
| 218.63 | 10.86 | 95.76 | 93.77 | 9.75 |
| 235.38 | 10.55 | 96.30 | 93.80 | 9.53 |
| 242.38 | 10.61 | 96.57 | 93.84 | 9.61 |

The P-modified ZSM-5 catalyst used in examples 1-7 deactivated during the toluene methylation reaction. The catalyst deactivation resulted in a decrease of yield of p-xylene with time on stream as shown in Tables 1-7. However, the decrease of p-xylene is dependent on conditions at which feed components were introduced into the reactor. For comparison, yields of p-xylene at 166-171 hour time are taken from examples 1-7 (Tables 1-7) and are shown in Table 8. Apparently, by maintaining the flow of hydrogen, optionally with steam and toluene, while increasing the reactor temperature to an elevated or reaction temperature and then introducing toluene and methanol produces improved p-xylene yield at a given time on stream (examples 5-7).

Table 8

| Example | Time on stream (h) | Toluene Conversion, mole% | Mixed Xylene Selectivity, mole% | Para-Xylene Selectivity, mole% | Para-Xylene Yield, mole% |
|---|---|---|---|---|---|
| Comparative 1 | 168 | 8.35 | 95.60 | 91.94 | 7.34 |
| Comparative 2 | 170 | 7.32 | 95.13 | 90.31 | 6.29 |
| Comparative 3 | 167 | 10.53 | 95.32 | 91.25 | 9.16 |
| Comparative 4 | 171 | 11.11 | 96.26 | 94.23 | 10.08 |
| 5 | 170 | 12.79 | 96.63 | 94.44 | 11.67 |
| 6 | 166 | 12.96 | 96.02 | 92.27 | 11.48 |
| 7 | 171 | 11.74 | 96.57 | 94.25 | 10.69 |

Obviously, numerous modifications and variations of the present invention are possible in light of the above teachings. It is therefore to be understood that within the scope of the appended claims, the invention may be practiced otherwise than as specifically described herein.

**Claims**

**1.** A method of alkylation of an aromatic comprising:

a) contacting an aromatic compound; an alkylating agent; hydrogen, an inert gas or a mixture thereof; and water with a shape-selective zeolite catalyst in a reactor in the steps of:

1) introducing hydrogen, an inert gas or a mixture thereof to the reactor at a reactor temperature of 200°C to less than 400°C; and
2) introducing an alkylating agent to the reactor at a reactor temperature of more than 400°C to 700°C;

b) withdrawing an alkylated aromatic from the reactor.

**2.** The method of Claim 1 wherein the hydrogen, inert gas or mixture thereof is introduced to the reactor at a reactor temperature in the range from 200°C to 250°C.

**3.** The method of Claim 1 wherein the alkylating agent is introduced to the reactor at a reactor temperature in the range from 400°C to 500° C.

**4.** The method of Claim 1 comprising:

contacting the aromatic compound, the alkylating agent; hydrogen, inert gas or a mixture thereof; and water with the shape-selective zeolite catalyst in a reactor in the steps of:

1) heating the reactor to a temperature of 200°C to less than 400°C;
2) introducing hydrogen, an inert gas or mixtures thereof to the reactor at a reactor temperature of 200°C to less than 400°C;
3) heating the reactor to a temperature of more than 400°C to 700°C; and
4) introducing an alkylating agent to the reactor at a reactor temperature of more than 400°C to 700°C;

wherein the aromatic compound is introduced to the reactor at step 2), step 3) or step 4) and water is introduced to the reactor at step 2), step 3) or step 4).

**5.** The method of Claim 4 wherein heating the reactor in step 1) is to a temperature in the range from w200°C to 250°C.

**6.** The method of Claim 5 wherein heating the reactor is by steaming the catalyst for 1 to 16 hours.

**7.** The method of Claim 4 wherein hydrogen is introduced to the reactor in step 1) while the reactor is being heated to a temperature of 200°C to less than 400°C.

**8.** The method of Claim 4 wherein the aromatic and the alkylating agent are mixed prior to being introduced into the reactor in step 4).

**9.** The method of Claim 4 wherein heating the reactor in step 3) is to a temperature in the range from 400°C to 500°C.

**10.** The method of claim 1 or 4 wherein the zeolite is a ZSM-5 zeolite.

**11.** The method of claim 1 or 4 wherein the aromatic compound is toluene.

**12.** The method of claim 1 or 4 wherein the aromatic compound is toluene and the alkylating agent is methanol.

**13.** The method of claim 12 wherein the toluene:methanol mole ratio is greater than 1:1, preferably wherein the toluene: methanol mole ratio is in the range from 2:1 to 20:1.

**14.** The method of claim 1 or 4 wherein the zeolite catalyst comprises a phosphorus-treated zeolite having a phosphorus content of from 0.01 to 0.15 gram of phosphorus per gram of zeolite.

**15.** The method of claim 4 wherein water and the aromatic are introduced to the reactor at step 4); or wherein water is introduced to the reactor at step 2) and the aromatic is introduced to the reactor at step 4); or wherein the aromatic and water are introduced to the reactor at step 2).

**Patentansprüche**

**1.** Verfahren zur Alkylierung eines Aromaten, das Folgendes umfasst:

a) Inberührungbringen von einer aromatischen Verbindung; einem Alkylierungsmittel; Wasserstoff, einem Inertgas oder einer Mischung davon und Wasser mit einem formselektiven Zeolithkatalysator in einem Reaktor in den folgenden Schritten:

1) Eintragen von Wasserstoff, einem Inertgas oder einer Mischung davon in den Reaktor bei einer Reaktortemperatur von 200°C bis weniger als 400°C und
2) Eintragen eines Alkylierungsmittels in den Reaktor bei einer Reaktortemperatur von mehr als 400°C bis 700°C;

b) Abziehen eines alkylierten Aromaten aus dem Reaktor.

**2.** Verfahren nach Anspruch 1, bei dem man den Wasserstoff, das Inertgas bzw. die Mischung davon bei einer Reaktortemperatur im Bereich von 200°C bis 250°C in den Reaktor einträgt.

**3.** Verfahren nach Anspruch 1, bei dem man das Alkylierungsmittel bei einer Reaktortemperatur im Bereich von 400°C bis 500°C in den Reaktor einträgt.

**4.** Verfahren nach Anspruch 1, das Folgendes umfasst:

Inberührungbringen von aromatischer Verbindung; Alkylierungsmittel; Wasserstoff, Inertgas oder einer Mischung davon und Wasser mit dem formselektiven Zeolithkatalysator in einem Reaktor in den folgenden Schritten:

1) Erhitzen des Reaktors auf eine Temperatur von 200°C bis weniger als 400°C;
2) Eintragen von Wasserstoff, einem Inertgas oder Mischungen davon in den Reaktor bei einer Reaktortemperatur von 200°C bis weniger als 400°C;
3) Erhitzen des Reaktors auf eine Temperatur von mehr als 400°C bis 700°C;
4) Eintragen eines Alkylierungsmittels in den Reaktor bei einer Reaktortemperatur von mehr als 400°C bis 700°C;

wobei die aromatische Verbindung in Schritt 2), Schritt 3) oder Schritt 4) in den Reaktor eingetragen wird und Wasser in Schritt 2), Schritt 3) oder Schritt 4) in den Reaktor eingetragen wird.

**5.** Verfahren nach Anspruch 4, bei dem der Reaktor in Schritt 1) auf eine Temperatur im Bereich von 200°C bis 250°C erhitzt wird.

**6.** Verfahren nach Anspruch 5, bei dem der Reaktor durch Bedampfen des Katalysators über einen Zeitraum von 1 bis 16 Stunden erhitzt wird.

**7.** Verfahren nach Anspruch 4, bei dem Wasserstoff in Schritt 1) in den Reaktor eingetragen wird, während der Reaktor auf eine Temperatur von 200°C bis weniger als 400°C erhitzt wird.

**8.** Verfahren nach Anspruch 4, wobei der Aromat und das Alkylierungsmittel vor dem Eintragen in den Reaktor in Schritt 4) gemischt werden.

**9.** Verfahren nach Anspruch 4, bei dem der Reaktor in Schritt 3) auf eine Temperatur im Bereich von 400°C bis 500°C erhitzt wird.

**10.** Verfahren nach Anspruch 1 oder 4, bei dem es sich bei dem Zeolithen um einen ZSM-5-Zeolithen handelt.

**11.** Verfahren nach Anspruch 1 oder 4, bei dem es sich bei der aromatischen Verbindung um Toluol handelt.

**12.** Verfahren nach Anspruch 1 oder 4, bei dem es sich bei der aromatischen Verbindung um Toluol handelt und es sich bei dem Alkylierungsmittel um Methanol handelt.

**13.** Verfahren nach Anspruch 12, bei dem das Toluol:Methanol-Molverhältnis größer als 1:1 ist und das Toluol:Methanol-Molverhältnis vorzugsweise im Bereich von 2:1 bis 20:1 liegt.

**14.** Verfahren nach Anspruch 1 oder 4, bei dem der Zeolithkatalysator einen mit Phosphor behandelten Zeolithen mit einem Phosphorgehalt von 0,01 bis 0,15 Gramm Phosphor pro Gramm Zeolith umfasst.

**15.** Verfahren nach Anspruch 4, bei dem Wasser und der Aromat in Schritt 4) in den Reaktor eingetragen werden oder bei dem Wasser in Schritt 2) in den Reaktor eingetragen wird und der Aromat in Schritt 4) in den Reaktor eingetragen wird oder bei dem der Aromat und Wasser in Schritt 2) in den Reaktor eingetragen werden.

## Revendications

**1.** Procédé d'alkylation d'un aromatique comprenant les étapes consistant à :

a) mettre en contact un composé aromatique ; un agent d'alkylation ; de l'hydrogène, un gaz inerte ou un mélange de ceux-ci ; et de l'eau avec un catalyseur de type zéolithe sélectif vis-à-vis de la forme dans un réacteur selon les étapes suivantes :

1) introduction d'hydrogène, d'un gaz inerte ou d'un mélange de ceux-ci dans le réacteur à une température du réacteur de 200°C à moins de 400°C ; et
2) introduction d'un agent d'alkylation dans le réacteur à une température du réacteur de plus de 400°C à 700°C ;

b) soutirage d'un aromatique alkylé du réacteur.

**2.** Procédé selon la revendication 1 dans lequel l'hydrogène, le gaz inerte ou le mélange de ceux-ci est introduit dans le réacteur à une température du réacteur dans la fourchette de 200°C à 250°C.

**3.** Procédé selon la revendication 1 dans lequel l'agent d'alkylation est introduit dans le réacteur à une température du réacteur dans la fourchette de 400°C à 500°C.

**4.** Procédé selon la revendication 1 comprenant les étapes consistant à :

mettre en contact le composé aromatique, l'agent d'alkylation, l'hydrogène, le gaz inerte, ou un mélange de ceux-ci, et de l'eau avec le catalyseur de type zéolithe sélectif vis-à-vis de la forme dans un réacteur selon les étapes suivantes :

1) chauffage du réacteur à une température de 200°C à moins de 400°C ;
2) introduction d'hydrogène, d'un gaz inerte ou mélanges de ceux-ci dans le réacteur à une température du réacteur de 200°C à moins de 400°C ;
3) chauffage du réacteur à une température de plus de 400°C à 700°C ; et
4) introduction d'un agent d'alkylation dans le réacteur à une température du réacteur de plus de 400°C à 700°C ;

dans lequel le composé aromatique est introduit dans le réacteur à l'étape 2), l'étape 3) ou l'étape 4) et l'eau est introduite dans le réacteur à l'étape 2), l'étape 3) ou l'étape 4).

**5.** Procédé selon la revendication 4 dans lequel le chauffage du réacteur dans l'étape 1) est fait à une température dans la fourchette de 200°C à 250°C.

**6.** Procédé selon la revendication 5 dans lequel le chauffage du réacteur est fait par traitement à la vapeur du catalyseur pendant 1 à 16 heures.

**7.** Procédé selon la revendication 4 dans lequel l'hydrogène est introduit dans le réacteur dans l'étape 1) tandis que le réacteur est chauffé à une température de 200°C à moins de 400°C.

**8.** Procédé selon la revendication 4 dans lequel l'aromatique et l'agent d'alkylation sont mélangés avant d'être introduits dans le réacteur dans l'étape 4).

**9.** Procédé selon la revendication 4 dans lequel le chauffage du réacteur dans l'étape 3) est fait à une température dans la fourchette de 400°C à 500°C.

**10.** Procédé selon la revendication 1 ou 4 dans lequel la zéolithe est une zéolithe ZSM-5.

**11.** Procédé selon la revendication 1 ou 4 dans lequel le composé aromatique est le toluène.

**12.** Procédé selon la revendication 1 ou 4 dans lequel le composé aromatique est le toluène et l'agent d'alkylation est le méthanol.

**13.** Procédé selon la revendication 12 dans lequel le rapport molaire toluène/méthanol est supérieur à 1:1, de préférence dans lequel le rapport molaire toluène/ méthanol est dans la fourchette de 2:1 à 20:1.

**14.** Procédé selon la revendication 1 ou 4 dans lequel le catalyseur de type zéolithe comprend une zéolithe traitée au phosphore ayant une teneur en phosphore de 0,01 à 0,15 gramme de phosphore par gramme de zéolithe.

**15.** Procédé selon la revendication 4 dans lequel l'eau et l'aromatique sont introduits dans le réacteur à l'étape 4) ; ou dans lequel l'eau est introduite dans le réacteur à l'étape 2) et l'aromatique est introduit dans le réacteur à l'étape 4) ; ou dans lequel l'aromatique et l'eau sont introduits dans le réacteur à l'étape 2).

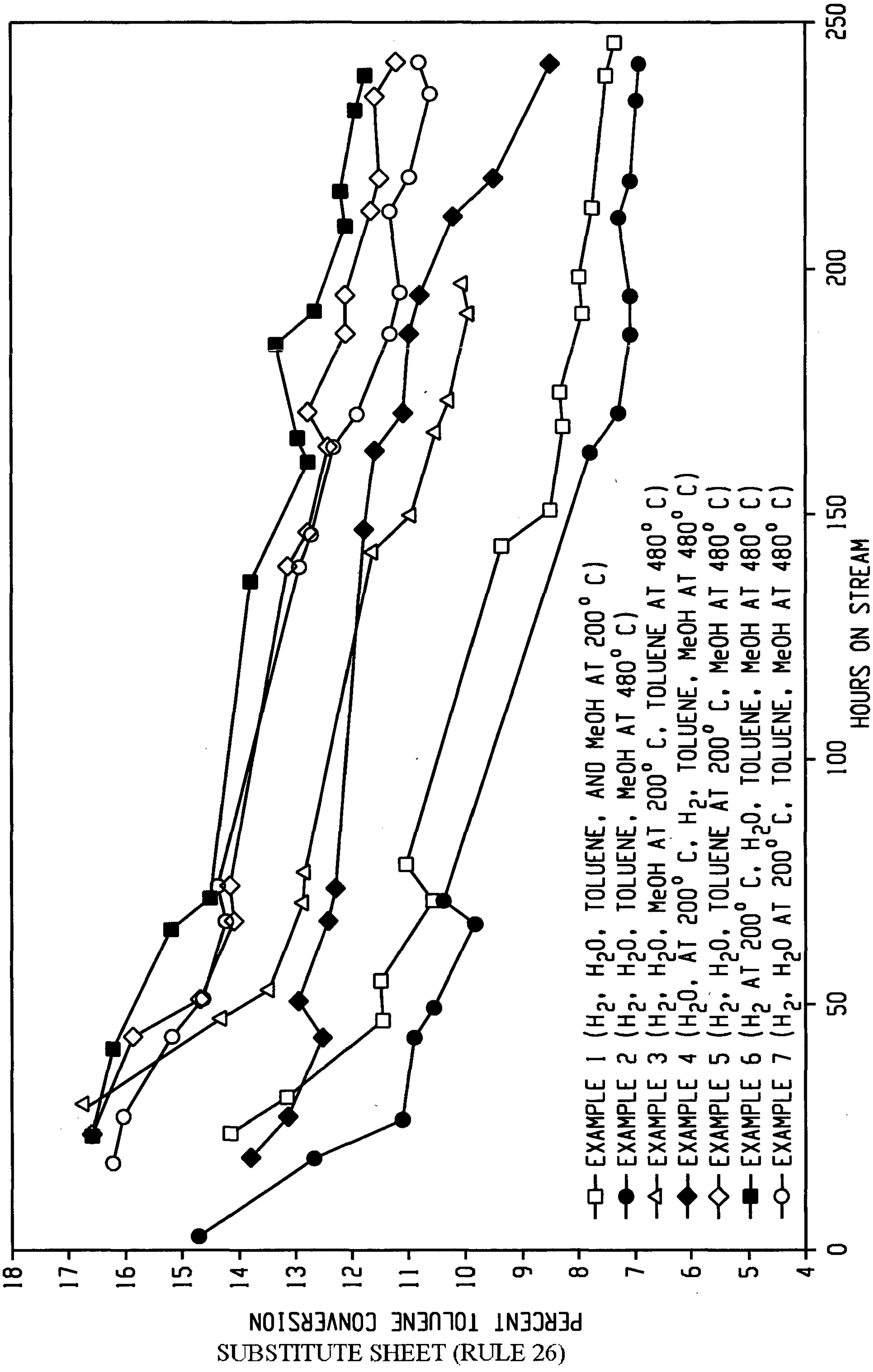


SUBSTITUTE SHEET (RULE 26)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4152364 A
- US 4250345 A
- US 7084318 B
- US 3702886 A
- US 7060864 B
- US 7186872 B


### Non-patent literature cited in the description


- Encarta® World English Dictionary. Microsoft Corporation, 2001